# EUROPÄISCHE PATENTSCHRIFT

(11) **EP 3 040 036 B1**
(45) Veröffentlichungstag und Bekanntmachung des Hinweises auf die Patenterteilung: **11.10.2017**
(21) Anmeldenummer: 14200435.7
(22) Anmeldetag: 29.12.2014
(51) Int. Cl.: A61M 5/168, A61B 17/3203, A61M 5/14, A61M 39/22, A61B 1/015, A61M 5/30, A61M 39/24, A61M 5/19, A61M 5/20, A61M 5/178, A61M 5/31

(54) **Instrumentenkopf, Applikationsinstrument mit entsprechendem Instrumentenkopf und Applikationssystem**
Instrument head, application instrument with corresponding instrument head and application system
Tête d'instrument, instrument d'application dotée de ladite tête d'instrument et système d'application

(43) Veröffentlichungstag der Anmeldung: 06.07.2016
(60) Teilanmeldung: 17158128.3
(73) Patentinhaber: Erbe Elektromedizin GmbH, 72072 Tübingen (DE)
(72) Erfinder: Fischer, Klaus, 72202 Nagold (DE); Enderle, Markus D., 72070 Tübingen (DE); Wandel, Waldemar, 72127 Kusterdingen (DE); Blobel, Lars, 72119 Ammerbuch-Entringen (DE); Fech, Andreas, 72072 Tübingen (DE)
(74) Vertreter: Bohnenberger, Johannes

(56) Entgegenhaltungen:
- EP-A1- 2 283 885
- WO-A1-2007/050553
- WO-A1-2013/171311
- US-A1- 2010 160 897

## Beschreibung

Die vorliegende Erfindung betrifft einen Instrumentenkopf, ein Applikationsinstrument mit entsprechendem Instrumentenkopf und ein Applikationssystem.

Entsprechende Applikationsinstrumente, die dazu geeignet sind, Substanzen oder Suspensionen, insbesondere Zellen, in ein biologisches Gewebe einzubringen, sind bekannt. Beispielsweise beschreibt die US 2001/0027296 A1 ein Applikationsinstrument, das Zellen aus einem Gewebe gewinnen kann, diese aufbereitet und dann wieder in das Gewebe einbringt.

Das Instrument der US 2011/0282381 A1 basiert im Wesentlichen darauf, dass ein entsprechender Kanal im Gewebe zum Einbringen der Substanzen bereits vorhanden ist. Gegebenenfalls kann ein entsprechender Kanal mit einer Spitze gestochen werden. Das Bereitstellen eines entsprechenden Kanals führt zu einer erheblichen Schädigung des zu behandelnden Gewebes. Des Weiteren ist es mit dem beschriebenen Instrument sehr schwierig, eine weiträumige und homogene Verteilung der einzubringenden Substanz zu erzielen.

Die WO 2013/171311 A1 beschreibt ein Abgabeinterface für zwei Flüssigkeiten mit einem Instrumentenkopf. Über zwei Zuläufe und ein flaches Ventilelement können Flüssigkeiten in einen Vorraum eingebracht werden und über eine Austrittsöffnung ausgebracht werden. Der Vorraum dient zum Mischen der Flüssigkeiten

Die EP 2 283 885 A1 beschreibt eine Dosiereinheit für eine Injektionsvorrichtung, mit einer Dosierkammer und zwei Zulaufleitungen. Um bei unterschiedlichem Druck in den Zulaufleitungen zu verhindern, dass Flüssigkeit in eine der Zulaufleitungen zurückfließt, können gemäß der D2 Rückschlagventile beim Übergang von der Dosierkammer zur Zulaufleitung angeordnet werden.

Die US 2010/160897 beschreibt eine Abgabeeinheit für die Abgabe eines therapeutischen Mittels. Über ein Ventil kann die Abgabe des therapeutischen Mittels gesteuert werden. Stromabwärts kann in einer Leitung ein Rückschlagventil angeordnet werden, welches verhindert, dass Flüssigkeit aus dem Speicher zurückfließt.

Die WO 2007/050553 A1 offenbart ein Ventil mit einer ersten Zulaufleitung und einer zweiten Zulaufleitung. Beim Einspritzen einer Flüssigkeit mit hohem Druck über die zweite Zulaufleitung wird eine flexible Zunge nach unten gedrückt, so dass der Zulauf über die erste Zulaufleitung unterbrochen wird.

Ausgehend von der US 2011/0282381 A1 ist es Aufgabe der vorliegenden Erfindung, einen Instrumentenkopf bereitzustellen, der ein effizientes Einbringen von Substanzen in biologisches Gewebe ermöglicht. Hierbei soll eine möglichst geringe Schädigung des Gewebes erfolgen und die Positionierung der eingebrachten Substanz optimiert werden. Des Weiteren soll nach Möglichkeit eine weiträumige und homogene Verteilung der eingebrachten Substanz erzielt werden, wobei die Substanz, bei der es sich beispielsweise um Zellen handeln kann, derart schonend behandelt wird, dass keine Schädigung der Substanz auftritt.

Diese Aufgabe wird durch den Instrumentenkopf gemäß Anspruch 1, das Applikationsinstrument gemäß Anspruch 6 und das Applikationssystem gemäß Anspruch 10 gelöst.

Ein Applikationsinstrument für ein Endoskop hat üblicherweise einen Instrumentengriff am proximalen Ende, einen vorzugsweise elastischen Schaft und einen Instrumentenkopf am distalen Ende. Erfindungsgemäß ist zumindest der Instrumentenkopf in besonderer Weise ausgestaltet.

Ein Kern der Erfindung besteht darin, die Substanzen, insbesondere eine (Zell-) Suspension möglichst effizient mit einem Hydrochirurgieinstrument
in das Gewebe einzubringen. Hierfür umfasst der erfindungsgemäße Instrumentenkopf einen Speicher, der die Substanz zwischenspeichern kann, bis diese über eine Düse appliziert wird. Die distale Anordnung (nahe der Spitze) des Speichers führt dazu, dass bei der Applikation von Druck auf die Substanz diese mehr oder weniger unmittelbar über eine Düse abgegeben werden kann. Insofern tritt nur ein sehr geringer Druckverlust auf. Außerdem wird die Einwirkdauer des Drucks deutlich reduziert. Dies führt dazu, dass die Substanz mit hohem Druck bei minimaler Belastung ausgebracht werden kann. Bei der Applikation von Zellen führt dies zu einer sehr hohen Überlebensrate, wodurch ein vielversprechendes Behandlungsergebnis erzielt werden kann.

Aufgrund des geringen Druckverlustes ist es auch möglich, die Substanz bei geringer Belastung oder Schädigung relativ tief in das Gewebe einzubringen. Die Applikation der Substanz mittels eines Wasserstrahl-Applikators hat allgemein den Vorteil, dass nur eine sehr geringe Schädigung des Zielgewebes eintritt und eine sehr gute Verteilung der Substanz erzielt werden kann.

Vorzugsweise umfasst der erfindungsgemäße Instrumentenkopf die erste Zulaufleitung und die zweite Zulaufleitung, wobei die Zulaufleitungen unterschiedliche Fluide führen. In einer Ausführungsform ist das erste Fluid ein Treibmittel und das zweite Fluid die zu applizierende Substanz oder Suspension. Die zweite Zulaufleitung kann also genutzt werden, um den Speicher mit der Substanz zu befüllen. Das Treibmittel wird über die erste Zulaufleitung in den Speicher eingeführt, so dass die Substanz aus diesem ausgetrieben wird. Das Einbringen kann aufgrund einer bestehenden fluiden Verbindung oder über ein Ventil erfolgen. Vorzugsweise ist dieses Ventil sehr nahe am distalen Ende angeordnet.

In einer Ausführungsform umfasst das mindestens eine Ventil mindestens ein Verschlussteil zum Verschließen der ersten Zulaufleitung gegenüber dem Speicher. Das Verschlussteil kann dazu dienen, beim Einbringen der Substanz zu verhindern, dass die erste Zulaufleitung zumindest teilweise mit der Substanz befüllt wird. Insofern kann verhindert werden, dass die "wertvolle" Substanz verlorengeht. Vorzugsweise ist das Ventil derart ausgebildet, dass in einem druckfreien Zustand die erste Zulaufleitung gegenüber dem Speicher verschlossen ist. Beispielsweise kann das Verschlussteil bzw. die Ventilmembran aus einem Elastomer ausgebildet sein. Vorzugsweise handelt es sich also um ein passives Ventil, so dass der notwendige Raum zur Anordnung des Ventils sehr gering ist. Dies hat den Vorteil, dass der Instrumentenkopf einen sehr geringen Durchmesser haben kann.

In einer Ausführungsform ist das mindestens eine Ventil ein Wechselventil, das entweder die erste Zulaufleitung oder die zweite Zulaufleitung gegenüber dem Speicher verschließt. Hierfür kann das Verschlussteil in entsprechender Weise angepasst sein. Bei der Applikation des ersten Fluids (beispielsweise des Treibmittels) kann so verhindert werden, dass dieses erste Fluid in den zweiten Zulauf bzw. die zweite Zulaufleitung eintritt. Insofern kann ein ungewolltes Vermischen des ersten und zweiten Fluids in der zweiten Zulaufleitung verhindert werden (Verwässerung der Substanz). Das Vorsehen des Wechselventils hat den weiteren Vorteil, dass das erste Fluid nicht nur als Treibmittel, sondern auch zum Herstellen eines Gewebekanals im biologischen Gewebe eingesetzt werden kann. Beispielsweise kann das erste Fluid zunächst gepulst ausgebracht werden, um den Kanal zu erstellen. Danach wird der Speicher mit dem zweiten Fluid gefüllt und wiederum mittels des ersten Fluids ausgebracht.

In dieser Konstellation hat das Wechselventil den weiteren Vorteil, dass das erste Fluid zum Herstellen des Gewebekanals mit wesentlich höherem Druck angetrieben werden kann, ohne dass es zu einer Schädigung der zu applizierenden Substanz kommt. Das Wechselventil schützt die Substanz vor einem zu hohen Druck, der gegebenenfalls einen negativen Einfluss auf die Wirkung der Substanz haben kann.

In der erfindungsgemäßen Ausführungsform umfasst die Austrittsöffnung ein Ventil, insbesondere in Form einer elastischen Düse. Ein elastischer Düsenkörper kann derart in den Instrumentenkopf eingebaut sein, dass eine definierte Pressung radial auf den Düsenkörper wirkt und im Ausgangszustand die Düsenöffnung verschlossen ist. Steigt der Druck im Speicher, z.B. infolge der Zufuhr des zweiten Fluids, so wird der Düsenkörper zunächst geringfügig nach außen gewölbt, ohne dass ein Austrittskanal geöffnet wird. Somit kann eine definierte Volumenmenge in dem Speicher vordosiert werden. Nachfolgend kann das vordosierte Volumen mittels des ersten Fluids ausgetrieben werden. Vorzugsweise ist die Materialhärte der elastischen Düse derart gewählt, dass eine ausreichende Dehnung und somit die Aufnahme eines Vordosierungsvolumens in dem Speicher erreicht wird. Hierfür ist vorzugsweise kein zu hoher Druck, beispielsweise größer als 20 bar, erforderlich.

Letztendlich kann eine elastische Düse auch das Verstopfen der Austrittsöffnung verhindern und sicherstellen, dass das zweite und/oder erste Fluid mit einem gewissen Minimaldruck ausgebracht wird.

In einer Ausführungsform ist im Instrumentenkopf ein weiteres Ventil zur Verhinderung eines Fluidrücklaufs in der zweiten Zulaufleitung vorgesehen. Hierbei kann es sich um ein Rückschlagventil handeln. Dieses zweite Ventil kann dazu dienen, einen voreingestellten Druck im Speicher, beispielsweise nach der Einbringung des zweiten Fluids, aufrechtzuerhalten. Des Weiteren verhindert das zweite Ventil ein Nachlaufen des zweiten Fluids, sobald das aktive Einbringen des zweiten Fluids in die zweite Zulaufleitung unterbunden wird. Insofern kann eine genauere Dosierung erfolgen.

Die eingangs genannte Aufgabe wird des Weiteren durch ein Applikationsinstrument für ein Endoskop gelöst, wobei das Applikationsinstrument vorzugsweise einen Schaft und einen Instrumentenhandgriff nahe dem oder am proximalen Ende des Schafts umfasst. Das Applikationsinstrument kann am proximalen Ende des elastischen Schafts mit dem bereits beschriebenen Instrumentenkopf in einer der beschriebenen Ausführungsformen ausgestattet sein.

Es ergeben sich ähnliche Vorteile, wie diese bereits in Verbindung mit dem Instrumentenkopf beschrieben wurden.

In einer Ausführungsform haben der Schaft und/oder das Instrument einen Außendurchmesser (Ad) von weniger als 3 mm. Mit der erfindungsgemäßen Ausführung des Instrumentenkopfs kann das erfindungsgemäße Ziel verwirklicht werden, wobei übliche Abmessungen von endoskopischen Instrumenten beibehalten werden können.

In einer Ausführungsform umfasst das Applikationsinstrument eine Belüftungseinrichtung in oder an der zweiten Zulaufleitung. Die Belüftungseinrichtung kann dazu ausgebildet sein, die zweite Zulaufleitung zu belüften. Vorzugsweise ist hierfür ein Wechselventil und evtl. ein weiterer kleiner Speicher vorgesehen. Ebenso kann eine Belüftungsöffnung vorgesehen sein, um ein Nachlaufen zu verhindern. Die Belüftungseinrichtung ermöglicht es, zumindest einen Teil der zweiten Zulaufleitung unmittelbar nach dem Einbringen des zweiten Fluids zu entlüften. Insofern kann die Menge des eingebrachten zweiten Fluids genau bemessen werden. Des Weiteren kann es die Belüftungseinrichtung ermöglichen, einen im Speicher aufgebauten Druck sehr schnell abzubauen. Es ist daher mit dem erfindungsgemäßen Applikationsinstrument möglich, die Fluide in gepulster Form abzugeben, wobei sehr steile Abfallflanken des Pulses erzielt werden. Dies ist die Voraussetzung für eine gepulste Abgabe der Fluide und einen schnellen Wechsel zwischen der Abgabe des ersten und/oder des zweiten Fluids. Des Weiteren erlaubt sie eine sehr gezielte Abgabe der Fluide. Beispielsweise kann hierdurch eine Dosierung der abgegebenen Fluide gewährleistet werden.

Der Instrumentengriff kann mindestens ein Stellventil oder Steuerventil mit einem Ventilantrieb umfassen, um eine gepulste/sequentielle Beaufschlagung der ersten Zulaufleitung und/oder der zweiten Zulaufleitung mit Druck zu generieren. Dieses Stellventil erleichtert die gepulste Fluidabgabe. Das Stellventil ermöglicht eine definierte Druckführung, wobei die Dehnung von Anschlussleitungen, beispielsweise zwischen dem Applikationsinstrument und einer Versorgungseinrichtung unberücksichtigt bleiben kann. Insofern können mit dem erfindungsgemäßen Applikationsinstrument deutlich bessere Ergebnisse erzielt werden. Diese verbesserten Ergebnisse werden durch die optimierte Form der Pulse, die Steilheit der Anstiegs- bzw. Abfallflanken erreicht.

Die eingangs genannte Aufgabe wird des Weiteren durch ein Applikationssystem gelöst. Das Applikationssystem umfasst vorzugsweise ein Applikationsinstrument wie es vorab beschrieben wurde. Das Applikationssystem kann des Weiteren eine Versorgungseinrichtung umfassen, die zumindest mit der ersten Zulaufleitung in fluider Verbindung steht.

Die Versorgungseinrichtung kann dazu ausgebildet sein, in einer Serie von Förderintervallen das erste Fluid in die erste Zulaufleitung zu fördern. Die Versorgungseinrichtung kann also zumindest das erste Fluid mit geeigneten Druckwerten bereitstellen. Auch für das Applikationssystem ergeben sich ähnliche Vorteile, wie diese bereits in Verbindung mit dem Applikationsinstrument beschrieben wurden.

Die Versorgungseinrichtung kann eine Steuerung umfassen, die mindestens ein Ventil, beispielsweise das bereits beschriebene Stellventil, derart steuert, dass innerhalb eines Applikationszeitintervalls von weniger als 2 Sekunden, insbesondere weniger als 1 Sekunde:
- das erste Fluid während mindestens eines ersten Förderintervalls mit einem ersten Druck in die erste Zulaufleitung;
- das zweite Fluid während eines zweiten, dem ersten Förderintervall zeitlich nachgelagerten Förderintervalls mit einem zweiten Druck in die zweite Zulaufleitung; und
- das erste Fluid während mindestens eines dritten Förderintervalls mit einem dritten Druck in die erste Zulaufleitung gefördert wird.

Erfindungsgemäß kann die Versorgungseinrichtung das erste und/oder zweite Fluid derart antreiben, dass innerhalb von kürzester Zeit das erste und das zweite Fluid, vorzugsweise im Wechsel, abgegeben wird. In einer bevorzugten Ausführungsform erfolgt eine unmittelbare Förderung des zweiten Fluids in die zweite Zulaufleitung nur dazu, um den bereits beschriebenen Speicher zu füllen. In dem genannten dritten Förderintervall wird dann wieder das erste Fluid gefördert, das als Treibmittel dient, um das zweite Fluid aus dem Speicher auszutreiben. Das applizierte dritte Pulsniveau (unter Entstehen des dritten Druckes) ist also maßgebend dafür, mit welchem Druck das zweite Fluid ausgegeben wird.

Die Nutzung des ersten Fluids nicht nur zur Freilegung eines Gewebekanals sondern auch als Treibmittel kann bei der Ausgestaltung des Applikationsinstruments vorteilhaft genutzt werden. Beispielsweise kann die erste Zulaufleitung deutlich druckfester ausgestaltet werden als die zweite Zulaufleitung, die mit einem vorzugsweise deutlich geringeren zweiten Druck betrieben wird.

Das Applikationssystem kann eine Pumpe zur Förderung des ersten Fluids und eine Medium-Trenneinrichtung umfassen, die derart angeordnet und ausgebildet ist, dass auch in dem zweiten Förderintervall das erste Fluid das zweite Fluid antreibt. Vorzugsweise wird die Versorgungseinrichtung dazu genutzt, um das erste und das zweite Fluid in die erste bzw. zweite Zulaufleitung hinein zu fördern. Hierfür kann in der Versorgungseinrichtung oder zwischen dem Applikationsinstrument und der Versorgungseinrichtung eine Medium-Trenneinrichtung vorgesehen werden, die das zweite Fluid bereitstellt und über das erste Fluid angetrieben wird. Im Endeffekt kann also auch im zeitlich nachgelagerten zweiten Förderintervall die Förderung des ersten Fluids maßgeblich für das Antreiben des zweiten Fluids sein.

Alternativ kann die Medium-Trenneinrichtung als zweite Pumpe bzw. Fluidquelle ausgebildet sein beziehungsweise ersetzt werden. Dies bedeutet, dass die Förderung des zweiten Fluids in den bereits beschriebenen Speicher unabhängig des ersten Fluids erfolgen kann.

Der erste Druck kann größer sein als der dritte Druck. Vorzugsweise ist der erste Druck deutlich größer als der dritte Druck, insbesondere um mindestens 30%. In einer Ausführungsform kann der erste Druck um mindestens 100% oder sogar um mindestens 200% größer sein als der dritte Druck. Vorzugsweise ist der erste Druck derart ausgelegt, dass damit ein zumindest teilweises Trennen des Zielgewebes möglich ist. Der erste Druck dient dazu, einen Kanal zur Einbringung der Substanz zu schaffen. In einer Ausführungsform bewegt sich der erste Druck zwischen 40 und 100 bar, insbesondere zwischen 60 und 90 bar. Der dritte Druck kann demgegenüber beispielsweise im Bereich zwischen 1 und 40 bar, insbesondere zwischen 2 und 20 bar, liegen. Vorzugsweise ist der dritte Druck so gewählt, dass ein schonendes Einbringen der Substanz gewährleistet wird. Des Weiteren hängt die Wahl des dritten Drucks davon ab, wie tief die Substanz in das Gewebe eingebracht werden soll.

Nachfolgend wird die Erfindung anhand von mehreren Ausführungsbeispielen beschrieben. Hierbei zeigt:
- Fig. 1: eine schematische Darstellung des Schichtaufbaus eines Hohlorgans;
- Fig. 2A: eine schematische Darstellung eines Applikationsinstruments gemäß einer ersten Ausführungsform (nur Wechselventil) mit Fluidfluss aus einem Innenzufuhrkanal;
- Fig. 2B: eine schematische Darstellung des Applikationsinstruments gemäß der ersten Ausführungsform mit Fluidfluss aus einem Außenzufuhrkanal;
- Fig. 3A: eine schematische Darstellung eines Applikationsinstruments gemäß einer zweiten Ausführungsform (Wechselventil mit distalem Rückschlagventil) mit Fluidfluss aus dem Innenzufuhrkanal;
- Fig. 3B: eine schematische Darstellung des Applikationsinstruments gemäß der zweiten Ausführungsform mit Fluidfluss aus dem Außenzufuhrkanal;
- Fig. 4A: eine schematische Darstellung eines Instrumentenkopfs gemäß einer dritten Ausführungsform (elastische Düse) mit Fluidfluss aus dem Außenzufuhrkanal;
- Fig. 4B: eine schematische Darstellung des Instrumentenkopfs gemäß der dritten Ausführungsform mit Fluidfluss aus dem Innenzufuhrkanal;
- Fig. 5: einen Instrumentenkopf einer vierten Ausführungsform (ebenfalls elastische Düse mit Zellsuspensionszufuhr durch den Außenzufuhrkanal);
- Fig. 6A: eine schematische Darstellung eines Applikationsinstruments gemäß einer fünften Ausführungsform (Entlüftungseinrichtung) mit Fluidfluss aus dem Außenzufuhrkanal;
- Fig. 6B: eine schematische Darstellung des Applikationsinstruments gemäß der fünften Ausführungsform mit Fluidfluss aus dem Innenzufuhrkanal;
- Fig. 7A: eine schematische Darstellung eines Applikationsinstruments gemäß einer sechsten Ausführungsform (Wechselventil mit proximalem Rückschlagventil aus einem elastischen Element) mit Fluidfluss durch den Außenzufuhrkanal;
- Fig. 7B: eine Detailansicht des elastischen Elements aus Fig. 7A;
- Fig. 8A: eine schematische Darstellung einer ersten alternativen Ausführungsform eines Wechselventils an dem Innenzufuhrkanal;
- Fig. 8B: eine schematische Darstellung einer zweiten alternativen Ausführungsform eines Wechselventils an dem Innenzufuhrkanal;
- Fig. 8C: eine schematische Darstellung einer dritten alternativen Ausführungsform des Ventilsitzes des Wechselventils an dem Innenzufuhrkanal;
- Fig. 9A: eine schematische Darstellung eines Applikationsinstruments gemäß einer siebten Ausführungsform;
- Fig. 9B: eine Detailansicht des Instrumentenkopfs gemäß Fig. 9A;
- Fig. 10: eine schematische Darstellung einer Versorgungseinrichtung gemäß einem ersten Ausführungsbeispiel, wobei alle Steuerventile in die Versorgungseinrichtung integriert sind;
- Fig. 11: eine schematische Darstellung einer Versorgungseinrichtung gemäß einem zweiten Ausführungsbeispiel, wobei ein Steuerventil in das Applikationsinstrument integriert ist;
- Fig. 12: eine schematische Darstellung eines ersten alternativen Aufbaus zur Erzeugung eines gepulsten Wasserstrahls;
- Fig. 13: eine schematische Darstellung eines zweiten alternativen Aufbaus zur Erzeugung eines gepulsten Wasserstrahls;
- Fig. 14: eine schematische Darstellung eines dritten alternativen Aufbaus zur Erzeugung eines gepulsten Wasserstrahls;
- Fig. 15: eine schematische Darstellung eines vierten alternativen Aufbaus zur Erzeugung eines gepulsten Wasserstrahls;
- Fig. 16: eine schematische Darstellung eines fünften alternativen Aufbaus zur Erzeugung eines gepulsten Wasserstrahls;
- Fig. 17: einen durch eine Versorgungseinrichtung generierten Druckverlauf gemäß einem ersten Steueralgorithmus;
- Fig. 18: einen durch eine Versorgungseinrichtung generierten Druckverlauf gemäß einem zweiten Steueralgorithmus;
- Fig. 19: den Druckverlauf gemäß Fig. 18 mit zusätzlich angezeigten Bypass- und Entlüftungsphasen.

In der nachfolgenden Beschreibung werden für gleiche und gleich wirkende Teile dieselben Bezugsziffern verwendet.

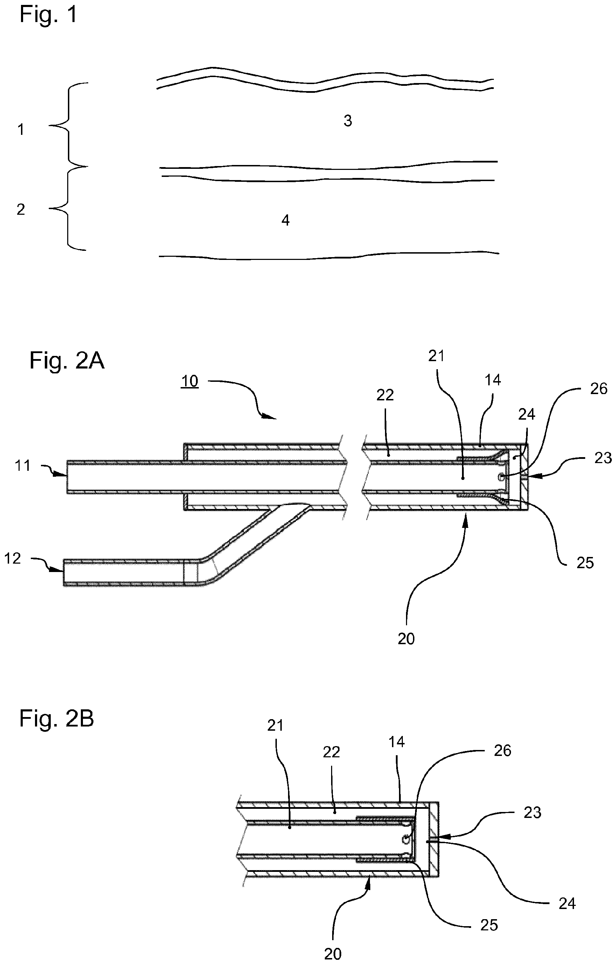

Fig. 1 zeigt eine schematische Darstellung des Schichtaufbaus eines Hohlorgans der ableitenden Harnwege. Wesentliche Gewebeschichten sind die Mucosa 1 und die Muscularis 2. Der Harnweg befindet sich in der Darstellung ganz oben. Danach folgt ein Epithel, das wiederum von der Lamina propria 3 gefolgt ist. Danach sind die Längsmuskel und Zirkulärmuskel 4 dargestellt. Das erfindungsgemäße Applikationssystem kann eingesetzt werden, um eine schnellere Regeneration eines Sphinkter-Defekts des dargestellten Harntrakts zu gewährleisten.

Das Applikationssystem ermöglicht eine tissue-engineering-basierte Therapie, bei der eine Suspension, beispielsweise Zellen in einem Nährmedium, in den Urethra-Schließmuskel durch mehrere vorgelagerte Gewebeschichten hindurch mit ausreichend hoher Überlebensrate der Zellen transportiert und dort möglichst verlustarm deponiert werden. Idealerweise wird hierbei eine Schädigung des noch intakten Schließmuskelgewebes verhindert. Der Zirkulärmuskel 4 aus Fig. 1 stellt daher ein mögliches Zielgewebe für das erfindungsgemäße Applikationssystem dar, wobei der applizierte Wasserstrahl zunächst die Mucosa 1 perforieren muss, um die Substanz zur Muscularis 2 zu transportieren.

Es gibt zahlreiche alternative Einsatzgebiete für das erfindungsgemäße Applikationssystem, beispielsweise Gallenwege, Gastrointestinalwände, Gefäßwände, Bronchialwände usw.

Fig. 2 zeigt ein erstes Ausführungsbeispiel eines erfindungsgemäßen Applikationsinstruments 10. Ein wesentlicher Bestandteil des Applikationsinstruments 10 ist der Sondenschaft 14, der vorzugsweise zumindest teilweise elastisch ist und proximal einen Instrumentenkopf 20 aufweist. Dieser Instrumentenkopf 20 hat eine Düse 23 zur Abgabe von Fluiden. Bei den Fluiden kann es sich um eine Kochsalzlösung handeln oder um die bereits erwähnte Suspension mit Zellanteilen. Um die Fluide zuführen zu können, ist im Lumen des Sondenschafts 14 koaxial ein Innenzufuhrkanal 21 angeordnet. Der Außenbereich des Innenzufuhrkanals 21 bildet den Außenzufuhrkanal 22, der den Innenzufuhrkanal 21 umgibt. Der Innenzufuhrkanal 21 steht in dem Zustand, wie er in Fig. 2A gezeigt ist, über Seitenöffnungen 26 in fluider Verbindung mit einem Distalspeicher 24. Ein an der distalen Spitze des Innenzufuhrkanals 21 angeordnetes Wechselventil 25 ermöglicht das Austreten eines ersten Fluids aus dem Innenkanal 21 in den Distalspeicher 24 und verschließt eine fluide Verbindung zwischen dem Distalspeicher 24 und dem Außenzufuhrkanal 22.

Der Innenzufuhrkanal 21 wird über einen ersten Zulauf 11, und der Außenzufuhrkanal 22 über einen zweiten Zulauf 12 mit dem ersten bzw. zweiten Fluid versorgt.

Fig. 2B zeigt eine Detailansicht des Instrumentenkopfs 20 aus Fig. 2A. Im Gegensatz zu der Darstellung gemäß Fig. 2A verschließt das Wechselventil 25 in Fig. 2B die Seitenöffnungen 26, so dass eine unmittelbare fluide Verbindung zwischen dem Distalspeicher 24 und dem Außenzufuhrkanal 22 besteht.

Ein Aspekt der vorliegenden Erfindung besteht darin, die zugeführten Fluide in einer annähernd perfekten Pulsform über eine Austrittsöffnung 23, die Düse 23 abzugeben. Der erfindungsgemäße Instrumentenkopf 20 ermöglicht es bei relativ niedrigen Drücken, Fluidpulse abzugeben, mit denen die Fluide in geeigneter Weise in das Zielgewebe eindringen können. Aufgrund der effizienten Nutzung der vorhandenen Drücke wird die Zellsuspension bei der Applikation "geschont".

Ein weiterer Aspekt der Erfindung besteht darin, mittels der Steuerung der Pulse die Fluide, insbesondere die Zellsuspension in unterschiedliche Ebenen des Zielgewebes einzubringen. Aufgrund der effizienten Nutzung der vorhandenen Drücke kann die Zellsuspension bei der Applikation "geschont" an das Zielgewebe insbesondere an verschiedene Stellen eingebracht werden.

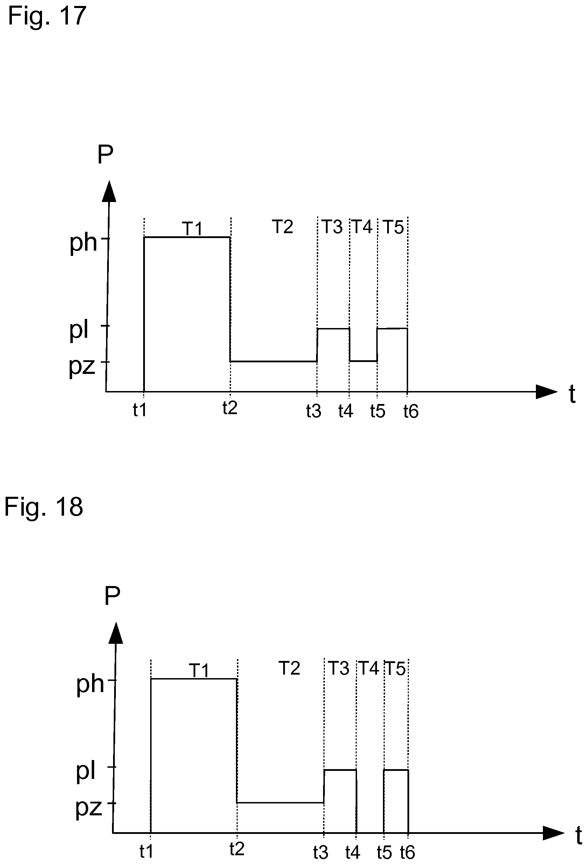

Für die effektive Einbringung der Suspension wird in einem Applikationszeitintervall, beispielsweise wie in Fig. 17 gezeigt, das erste Fluid zunächst in einem ersten Zeitintervall T1 mit einem hohen Druck ph gefördert. In diesem ersten Zeitintervall T1 befindet sich der Instrumentenkopf 20 in dem Zustand, wie er in Fig. 2A gezeigt ist. Das erste Fluid tritt aus dem Innenzufuhrkanal 21 aus, füllt den Distalspeicher 24 und wird über die Düse 23 mit einem definierten Düsendurchmesser abgegeben. Das erste Fluid trifft also mit einer hohen kinetischen Energie auf das Gewebe und kann genutzt werden, um einen Einbringungskanal zu schaffen. In einem nachfolgenden zweiten Zeitintervall T2 wird das zweite Fluid mit einem sehr niedrigen Druck pz angetrieben, so dass sich der Distalspeicher 24 mit dem zweiten Fluid - also der Suspension - füllt. In dieser Phase kann der Instrumentenkopf 20 den Zustand einnehmen, wie dieser in Fig. 2B gezeigt ist. Das Wechselventil 25 verschließt den Innenzufuhrkanal 21, so dass das Nachlaufen des ersten Fluids verhindert wird. Nach der Füllung des Distalspeichers 24 wird das erste Fluid in dem dritten Zeitintervall T3 mit einem Druck pl gefördert. Dieser Druck pl ist vorzugsweise deutlich geringer als der hohe Druck ph, so dass eine schonende Ausbringung der Suspension erfolgt. In dem dritten Zeitintervall T3 nimmt der Instrumentenkopf 20 wieder den Zustand ein, wie dieser in Fig. 2A gezeigt ist. Das erste Fluid dringt in den Distalspeicher 24 ein und verdrängt das zweite Fluid. Das erste Fluid ist also Treibmittel und dient zum Austreiben des zweiten Fluids bei gegebenem Druck pz. Je nach Ausgestaltung kann der Distalspeicher 24 in einem Applikationszeitintervall ein weiteres Mal gefüllt werden (vgl. viertes Zeitintervall T4) und die Suspension ein weiteres Mal abgegeben werden (vgl. Zeitintervall T5).

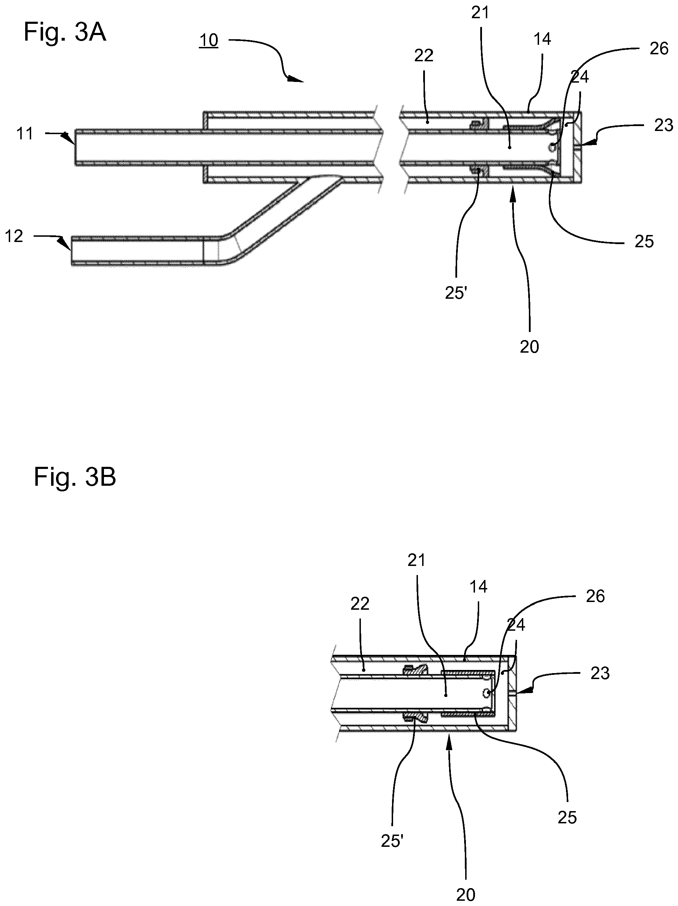

Fig. 3A zeigt eine weitere Ausführungsform des erfindungsgemäßen Applikationsinstruments 10. Gegenüber der Ausführungsform aus den Fig. 2A und 2B ist in der Ausführungsform gemäß Fig. 3A ein weiteres Ventil im Instrumentenkopf 20 vorgesehen. Das weitere Ventil, ein Rückschlagventil 25', befindet sich ebenso wie das Wechselventil 25, im Außenzufuhrkanal 22. Gegenüber dem Wechselventil 25 ist das Rückschlagventil 25' weniger nahe an der distalen Spitze des Applikationsinstruments 10 angeordnet. Bei dem Rückschlagventil 25' handelt es sich um eine Gummilippe, die im druckfreien Zustand den Außenzufuhrkanal 22 vollständig verschließt. Fig. 3A zeigt einen entsprechenden druckfreien Zustand, bei dem das erste Fluid gefördert und über die Düse 23 ausgegeben wird.

Bei einer Förderung des zweiten Fluids über den zweiten Zulauf in dem Außenzufuhrkanal 22 öffnet sich das Rückschlagventil 25' und das Wechselventil 25 verschließt, wie bereits erläutert, die Seitenöffnungen 26. Ein entsprechender Zustand ist in Fig. 3B gezeigt. In diesem Zustand kann der Distalspeicher 24 befüllt werden. Sobald der Fluidfluss in dem Außenzufuhrkanal 22 zum Erliegen kommt, schließt sich das Rückschlagventil 25'. Insofern wird ein Nachlaufen des zweiten Fluids verhindert. Übersteigt der Druck in dem Innenzufuhrkanal 21 den Druck des Distalspeichers 24, öffnet sich das Wechselventil 25. Dies kann dazu führen, dass bei dem gepulsten Strahl energetisch eine sehr steile Anfangsflanke erzeugt werden kann.

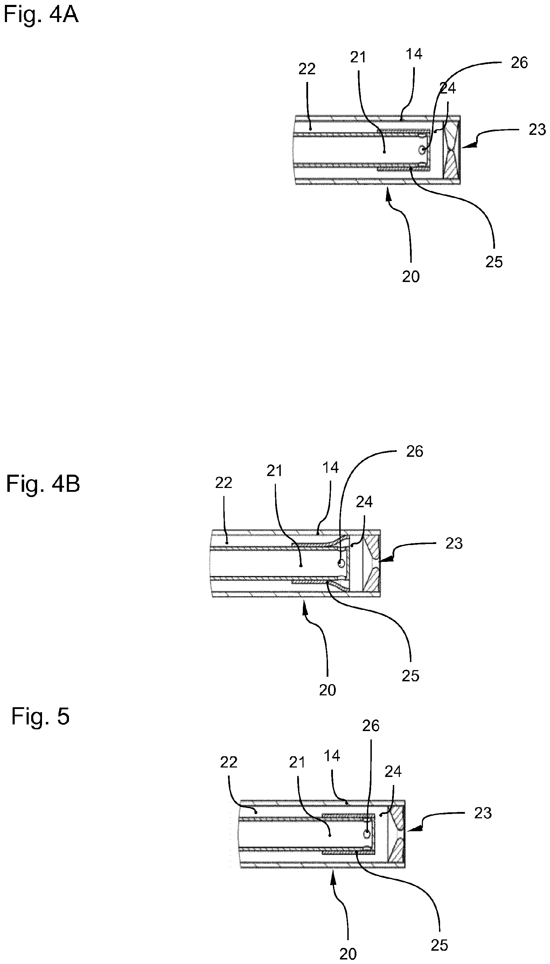

Die beschriebene Ausführungsform lässt sich in besonders vorteilhafter Weise mit einer elastischen Düse 23 einsetzen, wie diese exemplarisch in der Fig. 4A und 4B gezeigt ist. Die elastische Düse 23 kann aber auch unabhängig von einem vorhandenen oder nicht vorhandenen Rückschlagventil 25' Vorteile haben.

Die elastische Düse 23 gemäß Fig. 4A ist im druckfreien Zustand geschlossen. Hierzu wird der elastische Düsenkörper derart in das Applikationsinstrument 10 eingebaut, dass eine definierte Pressung radial auf den Düsenkörper wirkt und im Ausgangszustand die Düsenöffnung verschließt. Steigt der Druck proximal des Düsenkörpers im Distalspeicher 24, beispielsweise infolge der Zufuhr des zweiten Fluids, so wird der Düsenkörper zunächst geringfügig nach außen gewölbt, ohne dass die Düsenöffnung geöffnet wird (nicht gezeigt). Somit kann eine definierte Volumenmenge in dem Distalspeicher 24 vordosiert werden. Anschließend kann das vordosierte Volumen wie bereits beschrieben mit einem nachfolgenden Hochdruckpuls (vgl. drittes oder fünftes Zeitintervall T3, T5) in den im Gewebe geöffneten Kanal eingetragen werden. Der in diesem Ausführungsbeispiel beschriebene Düsenkörper besitzt eine umlaufende Lippe, die sich in Radialrichtung verjüngt. Der Düsenkörper kann auch zweigeteilt aufgebaut werden. Beispielsweise kann die umlaufende Lippe aus einem elastischen Material bestehen, während sie an ihrer Basis von einem Träger aus einem harten Material umfasst wird. Das Verhalten der elastischen Düse 23, insbesondere deren Dehnung, in der Vordosierungs- bzw. Befüllungsphase, hängt entscheidend von der Materialwahl und dem Ausmaß der Pressung im eingebauten Zustand ab. Erfindungsgemäß ist die elastische Düse 23 derart ausgestaltet, dass eine ausreichende Dehnung und somit die Aufnahme eines Vordosierungsvolumens im Distalspeicher 24 erreicht werden kann, ohne dass ein hoher Druck, beispielsweise höher als 20 bar erforderlich ist. Weiterhin ist die elastische Düse so ausgestaltet, dass im offenen Zustand die Düsenöffnung 23 gerade so groß ist, dass eine Düsenwirkung, also eine ausreichende Beschleunigung des Fluids, erzielt werden kann.

Die elastische Düse 23 kann erfindungsgemäß dazu eingesetzt werden, einen Nachlauf des Fluids nach der Applikation des ersten und/oder zweiten Fluids zu verhindern. Gleichzeitig speichert sie bei entsprechender Befüllung des Distalspeichers 24 einen gewissen Vordruck, der abgerufen werden kann. Weiterhin minimiert die elastische Düse 23 die Gefahr des Verstopfens des Applikationsinstruments 10. Die Verstopfung führt bei der erfindungsgemäßen Ausgestaltung lediglich zu einem Druckanstieg, der wiederum eine Dehnung der Düse 23 derart veranlasst, dass Verunreinigungspartikel passieren können.

Fig. 4B zeigt den Instrumentenkopf 20 mit geöffneter elastischer Düse 23, beispielsweise während des dritten Zeitintervalls T3.

Fig. 5 zeigt eine alternative Ausführungsform des Instrumentenkopfs gemäß Fig. 4A und 4B. Hier dient der Außenzufuhrkanal 22 zur Zufuhr des ersten Fluids und der Innenzufuhrkanal 21 zur Zufuhr des zweiten Fluids. Der in Fig. 5 gezeigte Zustand tritt beispielsweise im ersten Zeitintervall T1 auf, wenn das erste Fluid zur Schaffung eines Gewebekanals verwendet wird. Auch bei den anderen bereits beschriebenen Ausführungsformen kann erfindungsgemäß der Innenzufuhrkanal 21 für das zweite Fluid, und der Außenzufuhrkanal 22 für das erste Fluid verwendet werden.

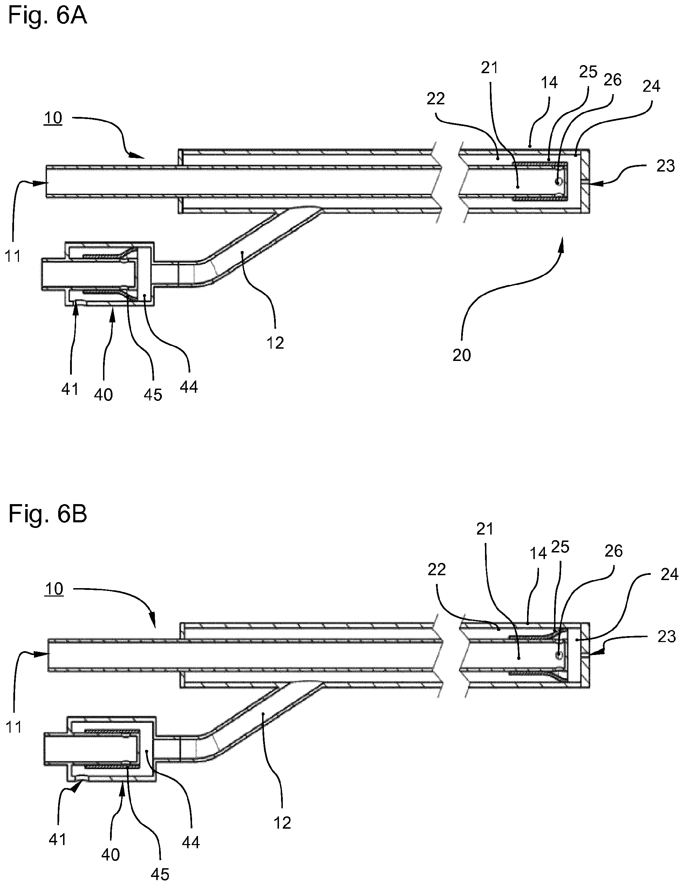

Die Fig. 6A und 6B zeigen ein weiteres erfindungsgemäßes Ausführungsbeispiel, bei dem ein elastisches Element als zweites passives Ventil verwendet wird. Im Unterschied zum Ausführungsbeispiel gemäß den Fig. 2A und 2B hat das Applikationsinstrument 10 der Fig. 6A im zweiten Zulauf eine Belüftungseinrichtung 40. Wesentliche Komponenten der Belüftungseinrichtung 40 sind eine Belüftungskammer 44, in die der zweite Zulauf 12 mündet, eine Belüftungsöffnung 41 und ein Belüftungsventil 45. Die Funktion der Belüftungsöffnung 41 wird mittels des Belüftungsventils 45 gesteuert. Die Belüftungsöffnung 41 ermöglicht eine Entlüftung des zweiten Zulaufs 12 und somit zumindest eines Abschnitts des Außenzufuhrkanals 22. Wird der zweite Zulauf 12 mit Druck beaufschlagt, schließt das Belüftungsventil 45 nach retrograd ab (Dichtwirkung zwischen zweitem Zulauf 12 und Belüftungsöffnung 41). Distal des Belüftungsventils 45 herrscht in der Belüftungskammer 44 ein Überdruck. Folglich kann das zweite Fluid in Richtung auf den Instrumentenkopf zuströmen und abgegeben werden (beispielsweise während des dritten Zeitintervalls T3). Dieser Zustand ist in der Fig. 6A gezeigt.

Fällt der Druck in dem zweiten Zulauf 12 ab, so geht das Belüftungsventil 45 in seinen Ausgangszustand über und verschließt den proximalen Teil des zweiten Zulaufs 12 gegenüber der Belüftungskammer 44 (vgl. Zustand gemäß Fig. 6B). Gleichzeitig wird der Überdruck in dem distalen Bereich des zweiten Zulaufs 12 sehr schnell über die Belüftungsöffnung 41 abgebaut. Dies führt dazu, dass der Nachlauf aus der Düse 23 sehr schnell gestoppt, im Idealfall verhindert wird, da der Strömungswiderstand der Belüftungsöffnung 41 deutlich geringer ist als derjenige der Düse 23. Insofern kann die Druckflanke des abgegebenen und gepulsten Fluidstrahls sehr steil abfallen. Da das Wechselventil 25 in diesem Zustand den Außenzufuhrkanal 22 verschließt, entweichen nur sehr geringe Mengen des zweiten Fluids durch die Belüftungsöffnung 41. Erfindungsgemäß ist es denkbar, eine Vorrichtung zum Aufnehmen der aus der Belüftungsöffnung austretenden Substanz vorzusehen und die Substanz ggf. rückzugewinnen. Erfindungsgemäß können natürlich auch mehrere Belüftungsöffnungen 41 vorgesehen sein.

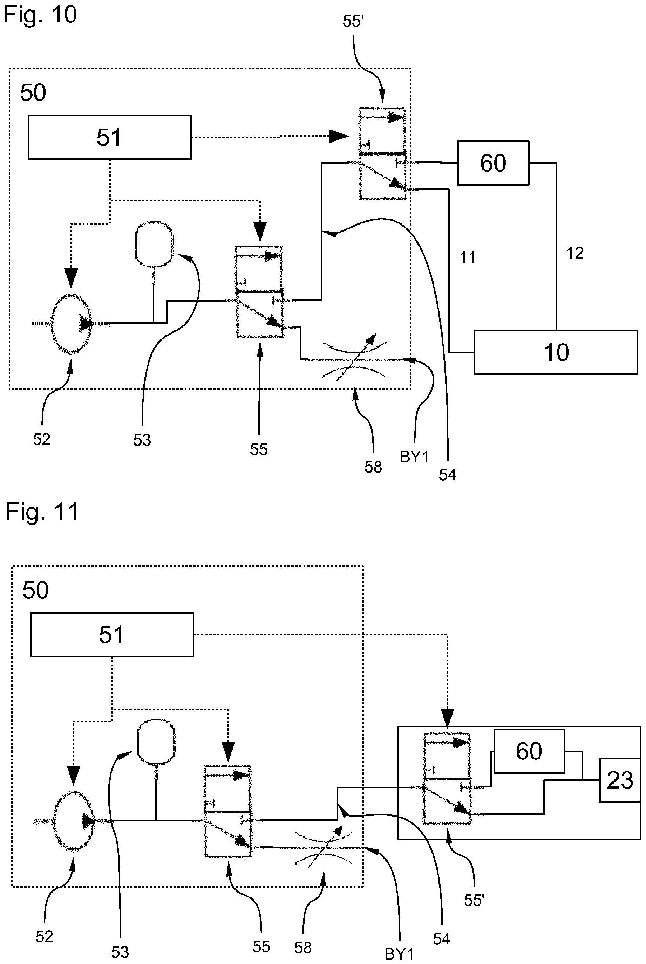

In einem anderen Ausführungsbeispiel handelt es sich bei dem Belüftungsventil 45 nicht um ein passives sondern um ein aktives Ventil bzw. ein Steuerventil. Beispielsweise kann im Handgriff des Applikationsinstruments 10 ein Magnetventil vorgesehen sein, das die Funktion des Belüftungsventils 45 erfüllt. Dieses Magnetventil kann von einer Versorgungseinrichtung 50 (vgl. Fig. 10) gesteuert werden.

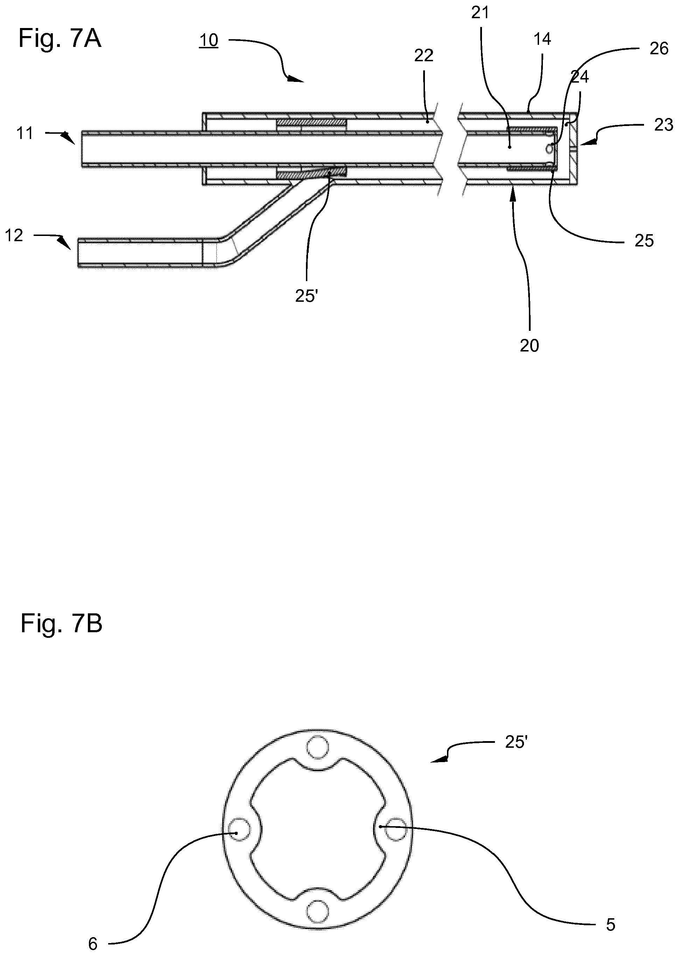

Die Fig. 7A zeigt eine Ausführungsform des Applikationsinstruments 10, die in ihrer Funktionsweise der der Fig. 3A und 3B ähnlich ist. Das Rückschlagventil 25' wird durch ein elastisches Element an einer Mündung des zweiten Zulaufs 12 in den Außenzufuhrkanal 22 gebildet. Das elastische Element ist derart ausgelegt, dass sich in einem nicht dargestellten Ausgangszustand eine Pressung zum Verschluss des zweiten Zulaufs 12 ergibt. Die dadurch erreichte Spannung erzeugt im drucklosen Zustand eine Dichtwirkung. Wird der zweite Zulauf 12 mit Druck beaufschlagt, verformt sich das elastische Element (vgl. Darstellung der Fig. 7A) und öffnet so die fluide Verbindung zu dem Außenzufuhrkanal 22. Nimmt der Druck in dem zweiten Zulauf 12 wieder ab, bringen die Rückstellkräfte das elastische Element nach Unterschreitung einer spezifischen Druckschwelle in den Ausgangszustand und das Rückschlagventil 25' schließt.

In einem Ausführungsbeispiel besteht das elastische Element aus einem elastischen Schlauchabschnitt. Durch das Anbringen von Verstärkungsstrukturen, wie beispielsweise den in Fig. 7B gezeigten in Richtung der Längsachse verlaufenden Rippen 5 und/oder Verstärkungsfasern 6 aus einem relativ steifen Material, kann eine höhere Anpressung und somit eine höhere Schließkraft des Rückschlagventils 25' erreicht werden.

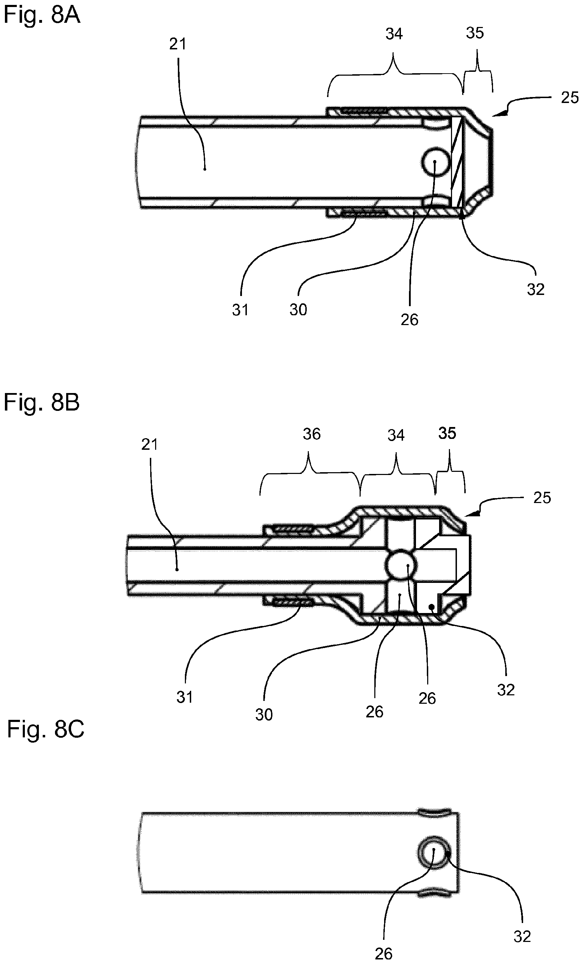

Die Fig. 8A, 8B, 8C zeigen unterschiedliche Ausführungsformen des Wechselventils 25. In einer bevorzugten Ausführungsform wird dieses durch elastisches Material ausgebildet, das an der Außenseite des Innenzufuhrkanals 21 angeordnet ist und diese mündende Seitenöffnungen 26 verschließt.

Die Ausführungsform gemäß Fig. 8A zeigt einen elastischen Schlauch 30, der abschnittsweise über das distale Ende des Innenzufuhrkanals 21 gestülpt ist. Eine Fixierung des elastischen Schlauches 30 erfolgt über einen Klemmring 31. Ein wesentlicher Aspekt der Ausführungsform gemäß Fig. 8A ist es, dass der elastische Schlauch 30 teilweise über das distale Ende des Innenzufuhrkanals 21 hinausragt. Im Endeffekt ergeben sich ein erster Abschnitt 34, an dem der elastische Schlauch auf dem Innenzufuhrkanal 21 aufliegt und ein zweiter Abschnitt 35, im Verlauf dessen sich der elastische Schlauch 30 verengt. Der elastische Schlauch 30 verläuft also über eine Dichtkante 32 mit einer besonders hohen Flächenpressung. Hierdurch wird eine bessere Dichtwirkung erzielt.

In der Ausführungsform gemäß Fig. 8B weist der Innenzufuhrkanal 21 abschnittsweise eine radial nach außen ragende Verdickung auf. Diese Verdickung ist an der Stelle angeordnet, an der sich auch die Seitenöffnungen 26 befinden. Proximal und distal der Verdickung hat der Innenzufuhrkanal 21 einen geringeren Durchmesser. Bezüglich des elastischen Schlauchs 30 ergibt sich also ein erster Abschnitt 34 mit größerem Durchmesser, ein zweiter Abschnitt 35 mit geringem Durchmesser und ein dritter Abschnitt 36 mit geringem Durchmesser. Die Durchmesser des zweiten und des dritten Abschnitts 35 und 36 können identisch sein. In einem anderen Ausführungsbeispiel (nicht dargestellt) können die Durchmesser des zweiten und des dritten Abschnitts 35 und 36 auch unterschiedliche Werte aufweisen. Durch verschiedenen Durchmesser des ersten Abschnitts 34 und des zweiten 35 und/oder des dritten Abschnitts 36 ergibt sich eine Dichtkante 32, die die Schließfunktion des Wechselventils 25 verbessert. Außerdem bewirkt der größere Durchmesser im ersten Abschnitt 34 eine Vordehnung und Vorspannung des elastischen Elements, was ebenfalls eine bessere Dichtwirkung bewirkt.

Eine höhere Flächenpressung kann auch durch Zylindersegmente erzielt werden, die die Seitenöffnungen 26 des Innenzufuhrkanals 21 umgeben (vgl. Fig. 8C). Im Endeffekt bilden diese Zylindersegmente auch Dichtkanten 32, die die Schließfunktion des Ventils verbessern.

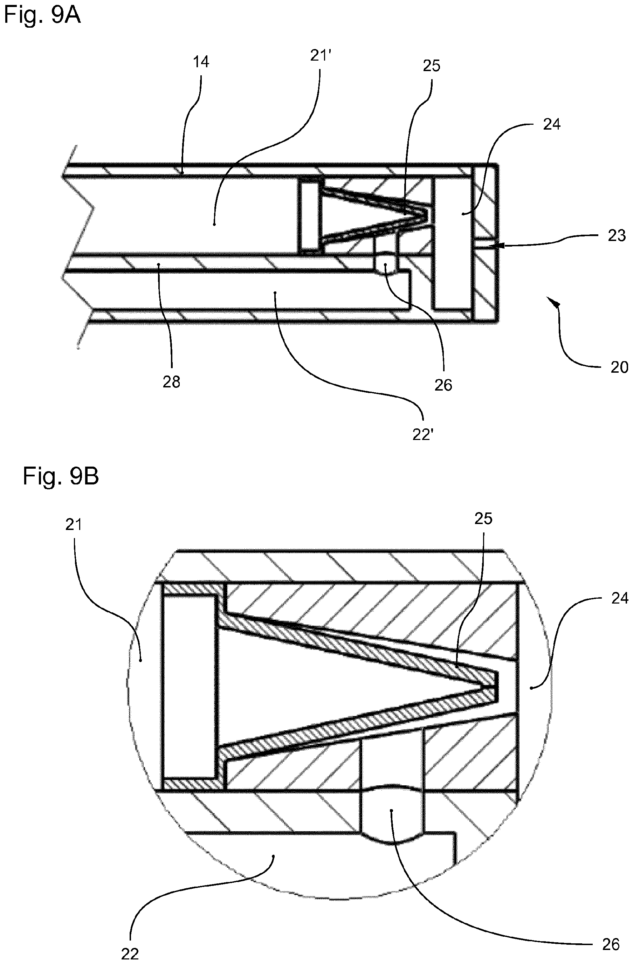

Die Fig. 9A und 9B zeigen weitere Ausführungsformen des Applikationsinstruments 10, insbesondere des Instrumentenkopfs 20. Der Sondenschaft 14 ist hier ein Mehrlumen-Schlauch (gezeigt ist ein zweilumiger Schlauch). In diesem Ausführungsbeispiel gibt es also keine koaxial zueinander angeordneten Kanäle, umfassend den Innenzufuhrkanal 21 und den Außenzufuhrkanal 22. Stattdessen verläuft ein erster Zufuhrkanal 21' für das erste Fluid parallel zu einem zweiten Zufuhrkanal 22'. Zwischen dem ersten Zufuhrkanal 21' und dem zweiten Zufuhrkanal 22' ist eine Trennwand 28 vorgesehen. In dem gezeigten Ausführungsbeispiel unterscheidet sich die Querschnittsfläche des ersten Zufuhrkanals 21' deutlich von der des zweiten Zufuhrkanals 22'. Beispielsweise kann der erste Zufuhrkanal 21' eine zweimal so große Querschnittsfläche wie der zweite Zufuhrkanal 22' haben. Diese sich deutlich unterscheidenden Querschnittsprofile können es gewährleisten, dass unterschiedlichen Volumenströmen (erfindungsgemäß ist der Volumenstrom des ersten Fluids größer als der des zweiten Fluids) Rechnung getragen werden. Erfindungsgemäß kann das Querschnittsprofil des ersten Zufuhrkanals 21' mehr als doppelt so groß sein als das Querschnittsprofil des zweiten Zufuhrkanals 22'.

Am distalen Ende verjüngt sich in der Ausführungsform gemäß Fig. 9A und 9B der erste Zufuhrkanal 21' und mündet dann in einen deutlich breiteren Distalspeicher 24, der in unmittelbarer fluider Verbindung mit der Düse 23 steht. Seitlich in dem verjüngenden Abschnitt des ersten Zufuhrkanals 21' mündet ungefähr mittig eine Seitenöffnung 26 des zweiten Zufuhrkanals 22'. In dem sich verjüngenden Abschnitt (= Halterungselement) ist ein elastisches Element zur Bildung eines Lippen-Rückschlagventils 25 angeordnet. Im Endeffekt ergibt sich hieraus ein bidirektionales Ventil, das die Funktion des bereits beschriebenen Wechselventils 25 übernimmt. Das Lippen-Rückschlagventil wird vorzugsweise in dem ersten Zufuhrkanal 21' durch das Halterungselement aus einem harten (nicht elastischen) Material axial fixiert. Die Innenkontur des sich verjüngenden Abschnitts entspricht weitgehend der Außenkontur des distalen Teils des Lippen-Rückschlagventils. Dabei ist die Öffnung des sich verjüngenden Abschnitts so dimensioniert, dass sich im Ausgangszustand (das elastische Element ist wie in Fig. 9A gezeigt, nicht gedehnt) ein Spalt zwischen den Lippen des elastischen Elements und der Innenwand des Halterungselements ergibt. Strömt z.B. das erste Fluid durch den ersten Zufuhrkanal 21', so wird der distale Abschnitt des elastischen Elements - die Lippen des Ventils - auseinandergedehnt, so dass es oberhalb einer Druckschwelle geöffnet wird, und das erste Fluid in den Distalspeicher 24 eintreten kann. Gleichzeitig wird die untere Ventillippe durch den Druck an die Wand des sich verjüngenden Abschnitts gedrückt. Somit schließt das elastische Element die Seitenöffnung 26 aus dem zweiten Zufuhrkanal 22'. Die Druckverhältnisse des ersten Zufuhrkanals 21' sind in diesem Zustand von den Druckverhältnissen des zweiten Zufuhrkanals 22' entkoppelt. Fällt der Druck in dem ersten Zufuhrkanal 21' ab (beispielsweise nach dem ersten Zeitintervall T1), so geht das Wechselventil 25 wieder in den Ausgangszustand über, so dass die Seitenöffnung 26, wie in der Fig. 9B gezeigt, freigegeben wird. Im zweiten Zeitintervall T2 kann das zweite Fluid durch den Spalt nahezu ungehindert vorbeiströmen, so dass ein Befüllen des Distalspeichers 24 bei geringem Druck möglich ist. Da das elastische Element nun in Sperrrichtung arbeitet, wirkt es wiederum als eine Barriere zwischen dem ersten Zufuhrkanal 21' und dem zweiten Zufuhrkanal 22'. Vorzugsweise ist der Spalt derart dimensioniert, dass das zweite Fluid ungehindert, bei geringem Druck strömen kann, und andererseits das Wechselventil 25 sicher geschlossen ist, um ein Eintreten des zweiten Fluids in den ersten Zufuhrkanal 21' zu verhindern.

Die gleiche Wirkung (bidirektionale Ventilwirkung) kann auch durch die Verwendung eines innenwirkenden Ventils in Kombination mit einem Kugelventil erreicht werden. Beide Ventile sind hintereinander in einem Lumen (vorzugsweise dem Größeren) angeordnet. Das Kugelventil befindet sich dabei proximal relativ zum elastischen Element. In dieser Anordnung übernimmt jedes der Ventile die Absperrung des Fluids in je eine Richtung, während der Fluss in der jeweils anderen Richtung ungehindert erfolgt.

Alle bisher beschriebenen Ausführungsformen haben das erfindungsgemäße Ziel, unterschiedliche Druckniveaus in dem ersten Zufuhrkanal 21' bzw. Innenzufuhrkanal 21 und dem zweiten Zufuhrkanal 22' bzw. Außenzufuhrkanal 22 zu erreichen. Dazu werden die Kanäle mit Hilfe von Ventilen voneinander entkoppelt. Gleichzeitig ermöglicht der Einsatz von passiven Ventilen in der beschriebenen Form ein Unterbinden des Nachlaufs sowie die Realisierung einer Druckspeicherfunktion. Diese beiden Funktionen sind vor allem in Kombination mit der Verwendung von proximal eingesetzten aktiven Ventilen besonders vorteilhaft. Nachfolgend werden mehrere erfindungsgemäße Versorgungseinrichtungen 50 zum Betreiben der beschriebenen Applikationsinstrumente beschrieben. Erfindungsgemäß können die Versorgungseinrichtungen 50 auch mit anderen Applikationsinstrumenten 10, beispielsweise herkömmlichen Applikationsinstrumenten, verwendet werden, um die nachfolgend beschriebenen vorteilhaften Effekte zu erzielen.

Fig. 10 zeigt eine Versorgungseinrichtung 50, die über den ersten Zulauf 11 und den zweiten Zulauf 12 mit einem der beschriebenen Applikationsinstrumente 10 verbunden ist. In dem in Fig. 10 gezeigten Ausführungsbeispiel ist in dem zweiten Zulauf eine Medium-Trenneinrichtung 60 vorgesehen, so dass die Versorgungseinrichtung über unterschiedliche Anschlüsse das erste Fluid, vorzugsweise eine Kochsalzlösung, abgeben kann, wobei je nach dem gewählten Zulauf letztendlich bei dem Applikationsinstrument 10 unterschiedliche Fluide ankommen (im ersten Zulauf 11 das erste Fluid und im zweiten Zulauf 12 das zweite Fluid).

Die Versorgungseinrichtung 50 umfasst eine Steuerung, die ein Steuerverfahren implementiert, bei dem innerhalb eines Applikationszeitintervalls folgende Schritte durchgeführt werden:
- Fördern des ersten Fluids während des ersten Förderintervalls T1 mit dem hohen Druck ph in die erste Zulaufleitung 11;
- mittelbares Fördern des zweiten Fluids während des zweiten Förderintervalls T2 mit dem zweiten Druck pz in der zweiten Zulaufleitung 12 unter Nutzung der Medium-Trenneinrichtung 60; und
- Fördern des ersten Fluids während des dritten Förderintervalls T3 mit dem dritten Druck pl in der ersten Zulaufleitung.

Erfindungsgemäß kann das Steuerverfahren dazu ausgebildet sein, um auch während des vierten Förderintervalls T4 und während des fünften Förderintervalls T5 eine entsprechende Steuerstrategie anzubieten (vgl. Fig. 17).

Zur Umsetzung des Steuerverfahrens interagiert die Steuerung 51 mit einer Fluidquelle beispielsweise einer Pumpe 52, einem ersten Steuerventil 55 und einem zweiten Steuerventil 55'.

Die Pumpe 52 steht in fluider Verbindung mit einem Druckspeicher 53 der Versorgungseinrichtung 50. In dem gezeigten Ausführungsbeispiel arbeitet die Pumpe 52 kontinuierlich und ist durchflussgeregelt. Die Steuerung des ersten Steuerventils 55 das in fluider Verbindung mit dem Druckspeicher 53 steht, ermöglicht es, eine gewünschte Pulsform (Frequenz, Tastgrad, effektive Pulsleistung) vorzugeben. Die Durchflussregelung der Pumpe bewirkt einen konstanten Volumenstrom des ersten Fluids innerhalb der Versorgungseinrichtung 50 unabhängig von der Schaltstellung des ersten Steuerventils 55.

Bei dem ersten Steuerventil 55 handelt es sich vorzugsweise um ein 3/2-Wegeventil, das im bestromten Zustand eine fluide Verbindung zwischen Druckspeicher 53 und einem zweiten Steuerventil 55' über eine Druckleitung 54 herstellt. Das erste Steuerventil 55 dient im Wesentlichen dazu, ein gewünschtes Druckniveau aufzubauen, während das zweite Steuerventil 55' das vorgegebene Druckniveau an den ersten Zulauf oder den zweiten Zulauf 12 anlegt.

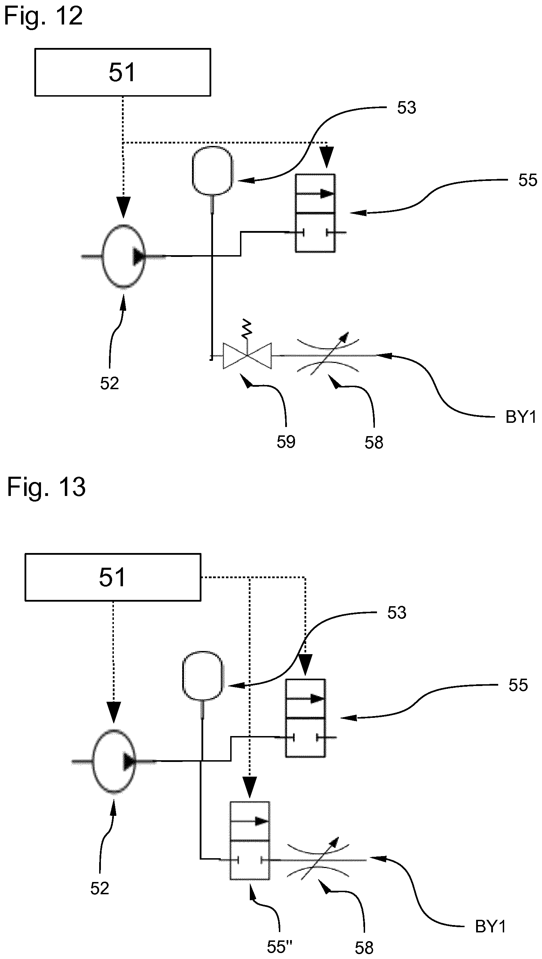

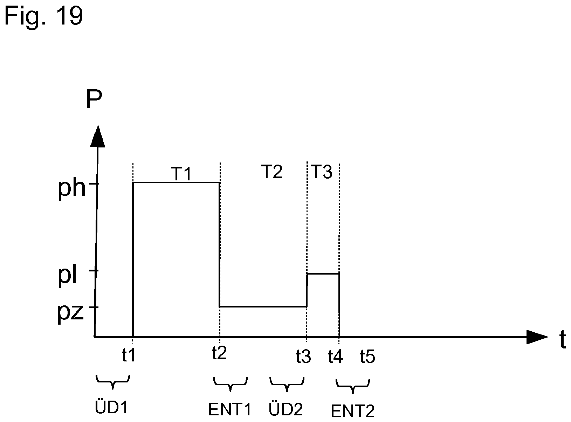

Im stromlosen Fall (vgl. Darstellung gemäß Fig. 10) des ersten Steuerventils 55 besteht eine fluide Verbindung zwischen dem Druckspeicher 53 - somit auch mit der Pumpe 52 - und einer ersten Bypassleitung BY1. Über die Bypassleitung BY1 wird der Fluidstrom abgeleitet, so dass kein unerlaubter Betriebszustand für die Pumpe 52 eintritt. Vorzugsweise ist die erste Bypassleitung BY1, wie in Fig. 10 gezeigt, mit einem Drosselventil 58 ausgestattet, das dafür sorgt, dass ein bestimmtes Druckniveau in dem Systemabschnitt vor dem ersten Steuerventil 55 gehalten wird. Zur Einstellung dieses Druckniveaus kann manuell oder über die Steuerung 51 (vgl. Fig. 13) ein hydraulischer Widerstand am Drosselventil 58 eingestellt werden. In dem in Fig. 10 gezeigten Ausführungsbeispiel ist der Widerstand voreingestellt. In dem in Fig. 10 gezeigten Zustand stellt sich in dem Druckspeicher 53 ein Druckniveau ein, das durch das Drosselventil 58 vorgegeben ist. Sobald dieses Druckniveau über das erste Steuerventil 55 an einen der beiden Zuläufe 11, 12 angelegt wird, steigt der Druck in dem jeweiligen Zulauf sprungartig an. Insofern kann ein Fluidpuls mit einer steilen Flanke abgegeben werden. Erfindungsgemäß ist es so möglich, während einer Bypassphase ÜD1, ÜD2 (vgl. Fig. 19) ein Druckniveau p'max zu erreichen, das über dem gewünschten Druck, beispielsweise dem ersten Druck ph oder dem dritten Druck pl, liegt. Diese Drücke werden vorzugsweise über die Förderleistung der Pumpe 52 eingestellt. Diese Drucküberhöhung führt dazu, dass sich der Druckimpuls sehr schnell in den Leitungen ausbreitet. Insofern kann an der Düse 23 eine sehr steile Pulsflanke erreicht werden. Des Weiteren kann die Drucküberhöhung Druckverluste in den Zuläufen 11, 12 kompensieren. Die Drucküberhöhung muss jedoch so gewählt werden, dass zwar ein schneller Kraftanstieg erreicht wird, der gewünschte Druck an der Düse 23 aber nicht überschritten wird.

Ausgehend von dem ersten Steuerventil 55 breitet sich der Druck im bestromten Zustand (nicht dargestellt) über die Druckleitungen 54 hin zu dem zweiten Steuerventil 55' aus. In dem beschriebenen Ausführungsbeispiel wählt das zweite Steuerventil 55' einen Zulauf 11, 12 aus.

In einem anderen Ausführungsbeispiel kann der Effekt der Drucküberhöhung genutzt werden, um eine initiale Perforation des biologischen Gewebes als vorbereitenden Schritt für den nachfolgenden Substanzeintrag durchzuführen. In diesem Ausführungsbeispiel generiert die Versorgungseinrichtung also ein steil ansteigendes Druckprofil, das über die Zeit abfällt. Das zweite Steuerventil 55' wird so geschaltet, dass über den Verlauf der abfallenden Druckflanke erst ein Perforieren des Gewebes (erstes Zeitintervall T1), dann ein Füllen des Distalspeichers 24 (zweites Zeitintervall T2) und letztendlich noch das Eintragen der Substanz (drittes Zeitintervall T3) erfolgt.

In einem weiteren Ausführungsbeispiel (vgl. Fig. 11) ist das zweite Steuerventil 55' in das Instrument 10 integriert. Vorzugsweise erfolgt eine Steuerung des zweiten Steuerventils 55' jedoch nach wie vor über die Steuerung 51. In einem weiteren bevorzugten Ausführungsbeispiel ist zusätzlich auch das erste Steuerventil 55 in das Applikationsinstrument integriert. Vorzugsweise erfolgt eine entsprechende Integration in einen Handgriff des Applikatorinstruments 10. Hierdurch kann vermieden werden, dass die Druckpulse durch lange und/oder elastische Zuleitungen gedämpft werden. Gemäß einem Aspekt der vorliegenden Erfindung erfolgt eine Anordnung der notwendigen Steuerventile 55, 55' möglichst nah am oder innerhalb des Applikationsinstruments 10.

In einer Ausführungsform wird die Anordnung des ersten Steuerventils 55 so gewählt, dass dieses im stromlosen Zustand die Verbindung zwischen der Druckleitung 54 und der Pumpe 52 absperrt. Die Druckleitung 54 ist also während der Bypassphasen ÜD1, ÜD2 druckfrei, bzw. mit einem Restdruck beaufschlagt. Diese Anordnung hat zwei Vorteile: Erstens muss das erste Steuerventil 55 nur während der Aktivierung zur Abgabe einer Pulsfolge jeweils nur kurzzeitig mit Strom beaufschlagt werden. Zweitens steht das durch das Drosselventil 58 eingestellte Druckniveau bereits beim ersten abgegebenen Puls zur Verfügung. Fig. 12 veranschaulicht eine andere Ausführungsform zur effektiven Generierung von Fluidpulsen. In dieser Ausführungsform wird als erstes Steuerventil 55 ein 2-Wegeventil eingesetzt. Wie auch in der vorab beschriebenen Ausführungsform besteht eine fluide Verbindung zwischen der Pumpe 52 und dem Druckspeicher 53. Des Weiteren besteht eine fluide Verbindung von dem Druckspeicher zu dem ersten Steuerventil 55 in Form eines 2/2-Wegeventils.

Des Weiteren gibt es eine fluide Verbindung von dem Druckspeicher 53 hin zu einem Überdruckventil 59 im ersten Bypass, dem ein Drosselventil 58 nachgeschaltet ist. Das 2/2-Wegeventil dient dazu, einen Wasserstrahl-Impuls mit definierter Dauer abzugeben, während das Überdruckventil 59 die Erzeugung eines gewünschten Druckniveaus während der Bypassphasen, ÜD1 ÜD2 ermöglicht. Dazu kann das Überdruckventil 59 so eingestellt werden, dass dieses beim Erreichen eines bestimmten Drucks die erste Bypassleitung freigibt, so dass sich der Druck abbauen kann. Das Überdruckventil 59 kann eine Regler-Funktion übernehmen, die vorzugsweise von der Steuerung 51 gesteuert wird. In einer anderen Ausführungsvariante kann die Druck-Druckfluss-Kennlinie des Überdruckventils 59 so ausgelegt werden, dass beim Durchströmen des Ventils ein gewisser Druck an dem Ventil abfällt. In einer Ausführungsform wird auf das Überdruckventil 59 gänzlich verzichtet.

Fig. 13 zeigt ein weiteres Ausführungsbeispiel, wobei zwei 2/2-Wegeventile eingesetzt werden. Im Endeffekt wird das erste Steuerventil 55 als 2/2-Wegeventil ausgelegt und dient nach wie vor dazu, die Weitergabe des in dem System bestehenden Drucks an das Applikationsinstrument 10 zu steuern. Ein drittes Steuerventil - ebenfalls ein 2/2-Wegenventil - erlaubt eine Druckeinstellung innerhalb des Systems indem es den ersten Bypass BY1 freigibt oder verschließt. Das dritte Steuerventil 55" kann maßgeblich dazu beitragen, einen gewissen Überdruck aufzubauen.

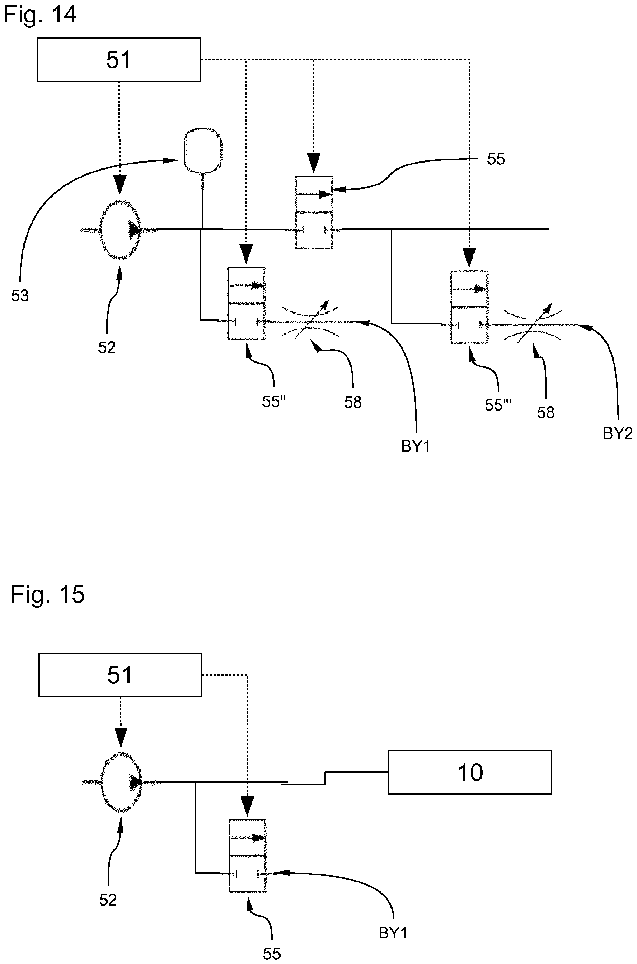

Um nach der Abgabe eines Impulses die Abfallszeit zu reduzieren, kann auf der flussabgewandten Seite des ersten Steuerventils eine weitere Bypassleitung BY2 vorgesehen sein. Fig. 14 zeigt eine entsprechende Ausführungsform der Ventilanordnung in einem Ausgangszustand wobei die Ventile entsprechend dem Applikationsschritt gesteuert werden. Ein viertes Steuerventil 55"' ermöglicht gezielt und schnell, den vorhandenen Druck in den Zuläufen 11, 12' abzubauen. Diese Ausführungsform ermöglicht es, Entlüftungsphasen ENT1, ENT2 umzusetzen, wie diese in der Fig. 19 gezeigt sind. Mit der in der Fig. 14 gezeigten Ausführungsform ist es also möglich, in einer ersten Bypassphase ÜD1 einen Überdruck aufzubauen und diesen dann gezielt an das Applikationsinstrument 10 abzugeben, so dass an dessen Düse 23 ein Puls mit einer möglichst steilen Flanke abgegeben wird. Danach treibt die Pumpe 52 das Fluid weiter an, um den ersten Druck während des ersten Zeitintervalls T1 aufrechtzuerhalten. Danach wird das erste Steuerventil 55 geschlossen, um den Puls zu beenden (Zeitpunkt t2). Gleichzeitig wird das vierte Steuerventil 55"' geöffnet, um eine fluide Verbindung zu der zweiten Bypassleitung BY2 zu schaffen. Hierdurch wird die erste Entlüftungsphase ENT1 implementiert. Die Abfallzeit ist sehr gering und führt zu einem unmittelbaren Druckabbau beispielsweise im Distalspeicher 24. In ähnlicher Weise kann verfahren werden, um während des dritten Zeitintervalls T3 - Applikation der Suspension - eine möglichst steile Pulsflanke am Anfang (Bypassphase ÜD2) und am Ende (Entlüftungsphase ENT2) zu erzielen.

Fig. 15 zeigt ein Ausführungsbeispiel, bei dem ein 2/2-Wegeventil als erstes Steuerventil 55 verwendet wird. Das 2/2-Wegeventil ist in die Bypassleitung BY1 integriert, während das Applikationsinstrument 10 direkt mit der Pumpe verbunden ist. Ein Vorteil dieses Aufbaus besteht darin, dass auch hier Entlüftungsphasen ENT1, ENT2 für einen schnellen Druckabfall implementiert werden können. Gegenüber den bisher beschriebenen Ausführungsformen ist diese Ausführungsform sehr einfach und robust.

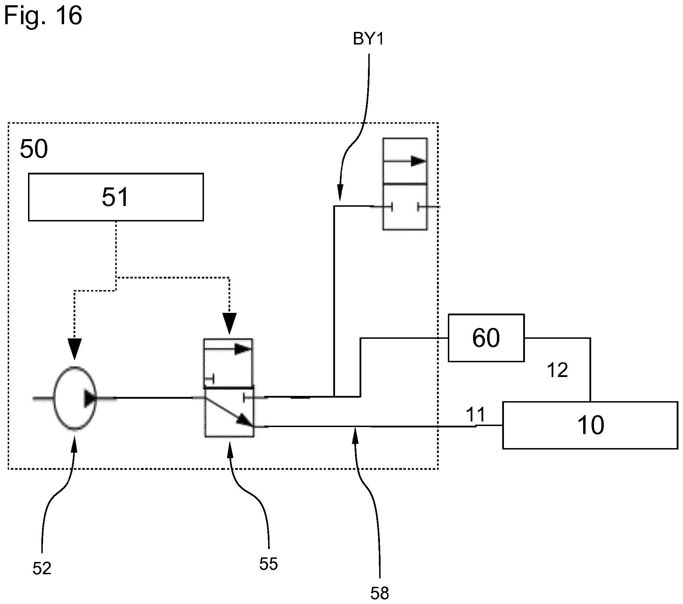

Fig. 16 zeigt ein weiteres Ausführungsbeispiel der erfindungsgemäßen Versorgungseinrichtung 50. Hier werden zwei 3/2-Wegeventile eingesetzt, um die bereits beschriebene Funktionalität umzusetzen.

Die beschriebenen aktiven Ventile bzw. Steuerventile können einen elektromagnetischen Antrieb oder einen anderen im Stand der Technik bekannten Antrieb besitzen. Beispielsweise können Piezo-Aktuatoren, eine pneumatische Antriebseinheit oder Ähnliches verwendet werden. Außerdem können die Ausführungsformen in beliebiger Weise miteinander kombiniert werden. Zur Umsetzung der Erfindung können Nadelventile, Membranventile, Wippenventile und andere eingesetzt werden. Zur Umsetzung des beschriebenen 2/2-Wegeventils kann beispielsweise ein Klemmventil eingesetzt werden, das aufgrund seiner Sterilisierbarkeit bevorzugt ist.

Fig. 17 und Fig. 18 zeigen unterschiedliche Druckverläufe, mit jeweils einem Puls für die Perforation des Gewebes (=erstes Zeitintervall T1) und zwei Pulsen für die Applikation der Substanz (=drittes und fünftes Zeitintervall T3 und T5). Das zweite Zeitintervall T2 wird jeweils genutzt, um den Distalspeicher 24 zu füllen. Gemäß Fig. 17 erfolgt ein Nachfüllen des Distalspeichers 24 in dem vierten Zeitintervall T4. Gemäß Fig. 18 unterbleibt ein entsprechendes Nachfüllen.

Fig. 19 zeigt die zeitliche Anordnung der Bypassphasen BY1, BY2 und der Entlüftungsphase ENT1, ENT2 relativ zu den Zeitintervallen T1 bis T5. Die erste Bypassphase endet mit dem Beginn des ersten Zeitintervalls T1 zum Zeitpunkt t1. Die erste Entlüftungsphase ENT1 beginnt am Ende des ersten Zeitintervalls T1 zum Zeitpunkt t2. Im Intervall zwischen t2 und t3 beginnt die zweite Bypassphase BY2, die zum Zeitpunkt t3 mit dem Beginn des dritten Zeitintervalls T3 endet. Am Ende des dritten Zeitintervalls T3 beginnt zum Zeitpunkt t4 die zweite Entlüftungsphase ENT2 die zum Zeitpunkt t5 endet.

### Bezugszeichenliste

- 1: Mucosa
- 2: Muscularis
- 3: Lamina propria
- 4: Circular muscule
- 5: Rippe
- 6: Verstärkungsfaser
- 10: Applikationsinstrument
- 11: Erster Zulauf, erste Zulaufleitung
- 12: Zweiter Zulauf, zweite Zulaufleitung
- 14: Sondenschaft
- 20: Instrumentenkopf
- 21: Innenzufuhrkanal
- 21': Erster Zufuhrkanal
- 22: Außenzufuhrkanal
- 22': Zweiter Zufuhrkanal
- 23: Austrittsöffnung, Düse
- 24: Distalspeicher
- 25: Wechselventil
- 25': Rückschlagventil
- 26: Seitenöffnung
- 28: Trennwand
- 30: Elastischer Schlauch
- 31: Klemmring
- 32: Dichtkante
- 34: Erster Abschnitt
- 35: Zweiter Abschnitt
- 36: Dritter Abschnitt
- 40: Belüftungseinrichtung
- 41: Belüftungsöffnung
- 44: Belüftungskammer
- 45: Belüftungsventil
- 50: Versorgungseinrichtung
- 51: Steuerung
- 52: Pumpe
- 53: Druckspeicher
- 54: Druckleitung
- 55, 55': Steuerventil
- 58: Drosselventil
- 59: Überdruckventil
- 60: Mediumtrenneinrichtung
- 100: Applikationssystem
- Ad: Außendurchmesser
- BY1, BY2: Bypassleitung
- ÜD1, ÜD2: Bypassphase
- ENT1, ENT2: Entlüftungsphase
- t1-t6: Zeitpunkt
- T1-T5: Zeitintervall
- pz, pl, ph: Druck

## Patentansprüche

1. Instrumentenkopf, umfassend:
- eine Austrittsöffnung (23);
- eine erste Zulaufleitung (11) zur Zuführung eines ersten Fluids;
- eine zweite Zulaufleitung (12) zur Zuführung eines zweiten Fluids;
- ein Speicher (24) zum Speichern des über die zweite Zulaufleitung (12) zugeführten Fluids;
wobei der Speicher (24) mit der ersten Zulaufleitung (11) in fluider Verbindung steht und/oder mit der ersten Zulaufleitung (11) über mindestens ein im Instrumentenkopf angeordnetes Ventil in fluide Verbindung bringbar ist, um das in dem Speicher (24) gespeicherte Fluid über die Austrittsöffnung (23) abzugeben,
**dadurch gekennzeichnet, dass**
die Austrittsöffnung (23) ein Ventil umfasst.

2. Instrumentenkopf nach Anspruch 1,
**gekennzeichnet durch**
mindestens ein Ventil (25, 25') mit mindestens einem Verschlussteil zum Verschluss der ersten Zulaufleitung (11) gegenüber dem Speicher (24), wobei das Verschlussteil zumindest teilweise aus einem Elastomer ausgebildet ist.

3. Instrumentenkopf nach einem der vorhergehenden Ansprüche,
**dadurch gekennzeichnet, dass**
das Ventil (25) ein Wechselventil ist, das entweder die erste Zulaufleitung (11) oder die zweite Zulaufleitung (12) gegenüber dem Speicher (24) verschließt.

4. Instrumentenkopf nach einem der vorhergehenden Ansprüche,
**dadurch gekennzeichnet, dass**
die Austrittsöffnung (23) eine elastische Düse mit einer umlaufenden Lippe umfasst, die sich in Radialrichtung verjüngt.

5. Instrumentenkopf nach einem der vorhergehenden Ansprüche,
**gekennzeichnet durch**
ein weiteres Ventil (25') in der zweiten Zulaufleitung (12), nämlich ein Rückschlagventil, zur Verhinderung eines Fluidrücklaufs in der zweiten Zulaufleitung (12).

6. Applikationsinstrument für ein Endoskop,
umfassend:
- einen elastischen Schaft (14);
- einen Instrumentenhandgriff nahe dem oder am proximalen Ende des Schafts (14),
**dadurch gekennzeichnet, dass**
am distalen Ende des elastischen Schafts (14) ein Instrumentenkopf (20) gemäß einem der vorhergehenden Ansprüche angeordnet ist.

7. Applikationsinstrument nach Anspruch 6,
**dadurch gekennzeichnet, dass**
der Schaft (14) und/oder der Instrumentenkopf (20) einen Außendurchmesser (Ad) von weniger als 3 mm hat.

8. Applikationsinstrument nach Anspruch 6 oder 7,
**gekennzeichnet durch**
eine Belüftungseinrichtung (40) in oder an der zweiten Zulaufleitung (12), wobei die Belüftungseinrichtung (40) ein Belüftungsventil (45) umfasst, um die zweite Zulaufleitung (12) zu belüften.

9. Applikationsinstrument nach einem der Ansprüche 6 bis 8,
**dadurch gekennzeichnet, dass**
der Instrumentengriff mindestens ein Steuerventil (55, 55') mit einem Ventilantrieb umfasst, um in der ersten Zulaufleitung (11) und/oder der zweiten Zulaufleitung (12) einen gepulsten Druck zu generieren.

10. Applikationssystem, umfassend:
- ein Applikatorinstrument (10) gemäß einem der Ansprüche 6 bis 8;
- eine Versorgungseinrichtung (50), die mit der ersten Zulaufleitung (11) in fluider Verbindung steht und dazu ausgebildet ist, in einer Serie von Förderintervallen (T1, T3, T5) das erste Fluid in die erste Zulaufleitung (11) zu fördern.

11. Applikationssystem nach Anspruch 10,
**dadurch gekennzeichnet, dass**
die Versorgungseinrichtung (50) eine Steuerung (51) umfasst, die mindestens ein Ventil (55) derart steuert, dass innerhalb eines Applikationszeitintervalls von weniger als 2 Sekunden:
- das erste Fluid während mindestens eines ersten Förderintervalls (T1) mit einem ersten Druck (ph) in die erste Zulaufleitung (11);
- das zweite Fluid während eines zweiten, dem ersten Förderintervall (T1) zeitlich nachgelagerten Förderintervalls (T2) mit einem zweiten Druck (pz) in die zweite Zulaufleitung (12); und
- das erste Fluid während mindestens eines dritten Förderintervalls (T3) mit einem dritten Druck (pl) in die erste Zulaufleitung gefördert wird.

12. Applikationssystem nach Anspruch 10 oder 11,
**gekennzeichnet durch**
eine Pumpe (52) zur Förderung des ersten Fluids und
eine Mediumtrenneinrichtung (60), die derart angeordnet und ausgebildet ist, dass in dem zweiten Förderintervall das erste Fluid das zweite Fluid antreibt.

13. Applikationssystem nach einem der Ansprüche 10 bis 12,
**dadurch gekennzeichnet, dass**
der erste Druck (ph) deutlich größer, nämlich um mindestens 30% oder um mindestens 100% oder um mindestens 200% des dritten Drucks (pl), als der dritte Druck (pl), ist.

## Claims

1. Instrument head comprising:
- an exit opening (23);
- a first inflow line (11) for feeding a first fluid;
- a second inflow line (12) for feeding a second fluid;
- a reservoir (24) for storing the fluid fed via the second inflow line (12);
wherein the reservoir (24) is in fluidic connection with the first inflow line (11) and/or can be brought into fluidic connection with the first inflow line (11) via at least one valve arranged in the instrument head, in order for the fluid stored in the reservoir (24) to be discharged via the exit opening (23),
**characterized in that**
the exit opening (23) comprises a valve.

2. Instrument head according to Claim 1,
**characterized by**
at least one valve (25, 25') having at least one closure part for closing the first inflow line (11) in relation to the reservoir (24), wherein the closure part is formed, at least in part, from an elastomer.

3. Instrument head according to either of the preceding claims,
**characterized in that**
the valve (25) is a shuttle valve which closes either the first inflow line (11) or the second inflow line (12) in relation to the reservoir (24).

4. Instrument head according to one of the preceding claims,
**characterized in that**
the exit opening (23) comprises an elastic nozzle with an encircling lip which tapers in the radial direction.

5. Instrument head according to one of the preceding claims,
**characterized by**
a further valve (25') in the second inflow line (12), that is to say a non-return valve, for preventing a backflow of fluid in the second inflow line (12).

6. Application instrument for an endoscope, comprising:
- an elastic shank (14);
- an instrument handle in the vicinity of, or at, the proximal end of the shank (14),
**characterized in that**
an instrument head (20) according to one of the preceding claims is arranged at the distal end of the elastic shank (14).

7. Application instrument according to Claim 6,
**characterized in that**
the shank (14) and/or the instrument head (20) have/has an external diameter (Ad) of less than 3 mm.

8. Application instrument according to Claim 6 or 7,
**characterized by**
a venting device (40) in or on the second inflow line (12), wherein the venting device (40) comprises a venting valve (45) in order to vent the second inflow line (12).

9. Application instrument according to one of Claims 6 to 8,
**characterized in that**
the instrument handle comprises at least one control valve (55, 55') with a valve drive, in order to generate a pulsed pressure in the first inflow line (11) and/or the second inflow line (12).

10. Application system comprising:
- an applicator instrument (10) according to one of Claims 6 to 8;
- a supply device (50) which is in fluidic connection with the first inflow line (11) and is designed to deliver the first fluid into the first inflow line (11) in a series of delivery intervals (T1, T3, T5).

11. Application system according to Claim 10,
**characterized in that**
the supply device (50) comprises a control means (51) which controls at least one valve (55) such that, within an application time interval of less than 2 seconds:
- the first fluid is delivered into the first inflow line (11) at a first pressure (ph) during at least a first delivery interval (T1);
- the second fluid is delivered into the second inflow line (12) at a second pressure (pz) during a second delivery interval (T2), which follows the first delivery interval (T1) in time; and
- the first fluid is delivered into the first inflow line at a third pressure (pl) during at least a third delivery interval (T3).

12. Application system according to Claim 10 or 11,
**characterized by**
a pump (52) for delivering the first fluid, and
a medium-separating device (60) which is arranged, and designed, such that the first fluid drives the second fluid in the second delivery interval.

13. Application system according to one of Claims 10 to 12,
**characterized in that**
the first pressure (ph) is considerably greater than the third pressure (pl), that is to say by at least 30% or by at least 100% or by at least 200% of the third pressure (pl).

## Revendications

1. Tête d'instrument, comprenant :
- une ouverture de sortie (23) ;
- une première conduite d'alimentation (11) pour acheminer un premier fluide ;
- une deuxième conduite d'alimentation (12) pour acheminer un deuxième fluide ;
- un accumulateur (24) pour accumuler le fluide acheminé par le biais de la deuxième conduite d'alimentation (12) ;
l'accumulateur (24) étant en liaison fluidique avec la première conduite d'alimentation (11) et/ou pouvant être amené en liaison fluidique avec la première conduite d'alimentation (11) par le biais d'au moins une soupape disposée dans la tête d'instrument, afin de délivrer le fluide accumulé dans l'accumulateur (24) par le biais de l'ouverture de sortie (23),
**caractérisée en ce que**
l'ouverture de sortie (23) comprend une soupape.

2. Tête d'instrument selon la revendication 1,
**caractérisée par**
au moins une soupape (25, 25') avec au moins une partie de fermeture pour la fermeture de la première conduite d'alimentation (11) par rapport à l'accumulateur (24), la partie de fermeture étant réalisée au moins en partie à partir d'un élastomère.

3. Tête d'instrument selon l'une quelconque des revendications précédentes,
**caractérisée en ce que**
la soupape (25) est une soupape de distribution qui ferme soit la première conduite d'alimentation (11) soit la deuxième conduite d'alimentation (12) par rapport à l'accumulateur (24).

4. Tête d'instrument selon l'une quelconque des revendications précédentes,
**caractérisée en ce que**
l'ouverture de sortie (23) comprend une buse élastique avec une lèvre périphérique qui se rétrécit dans la direction radiale.

5. Tête d'instrument selon l'une quelconque des revendications précédentes,
**caractérisée par**
une soupape supplémentaire (25') dans la deuxième conduite d'alimentation (12), à savoir un clapet antiretour, pour empêcher un reflux de fluide dans la deuxième conduite d'alimentation (12).

6. Instrument d'application pour un endoscope, comprenant :
- une tige élastique (14) ;
- une poignée d'instrument à proximité ou au niveau de l'extrémité proximale de la tige (14),
**caractérisé en ce**
**qu'**à l'extrémité distale de la tige élastique (14) est disposée une tête d'instrument (20) selon l'une quelconque des revendications précédentes.

7. Instrument d'application selon la revendication 6,
**caractérisé en ce que**
la tige (14) et/ou la tête d'instrument (20) présente un diamètre extérieur (Ad) inférieur à 3 mm.

8. Instrument d'application selon la revendication 6 ou 7,
**caractérisé par**
un dispositif de ventilation (40) dans ou sur la deuxième conduite d'alimentation (12), le dispositif de ventilation (40) comprenant une soupape de ventilation (45) afin de ventiler la deuxième conduite d'alimentation (12).

9. Instrument d'application selon l'une quelconque des revendications 6 à 8,
**caractérisé en ce que**
la poignée d'instrument comprend au moins une soupape de commande (55, 55') avec un entraînement de soupape afin de générer une pression pulsée dans la première conduite d'alimentation (11) et/ou la deuxième conduite d'alimentation (12).

10. Système d'application, comprenant :
- un instrument applicateur (10) selon l'une quelconque des revendications 6 à 8 ;
- un dispositif d'alimentation (50) qui est en liaison fluidique avec la première conduite d'alimentation (11) et qui est réalisé pour refouler le premier fluide dans la première conduite d'alimentation (11) dans une série d'intervalles de refoulement (T1, T3, T5).

11. Système d'application selon la revendication 10,
**caractérisé en ce que**
le dispositif d'alimentation (50) comprend une commande (51) qui commande au moins une soupape (55) de telle sorte que dans l'espace d'un intervalle d'application inférieur à 2 secondes :
- le premier fluide soit refoulé pendant au moins un premier intervalle de refoulement (T1) avec une première pression (ph) dans la première conduite d'alimentation (11) ;
- le deuxième fluide soit refoulé pendant un deuxième intervalle de refoulement (T2) décalé dans le temps après le premier intervalle de refoulement (T1) avec une deuxième pression (pz) dans la deuxième conduite d'alimentation (12) ; et
- le premier fluide soit refoulé pendant au moins un troisième intervalle de refoulement (T3) à une troisième pression (pl) dans la première conduite d'alimentation.

12. Système d'application selon la revendication 10 ou 11,
**caractérisé par**
une pompe (52) pour refouler le premier fluide et un dispositif de séparation de milieu (60) qui est disposé et réalisé de telle sorte que le premier fluide entraîne le deuxième fluide dans le deuxième intervalle de refoulement.

13. Système d'application selon l'une quelconque des revendications 10 à 12,
**caractérisé en ce que**
la première pression (ph) est nettement supérieure, à savoir d'au moins 30% ou d'au moins 100 % ou d'au moins 200 %, de la troisième pression (pl), à la troisième pression (pl).
